# EUROPEAN PATENT APPLICATION

(11) **EP 2 206 506 A1**
(43) Date of publication of application: **14.07.2010**
(21) Application number: 08172103.7
(22) Date of filing: 18.12.2008
(51) Int. Cl.: A61K 35/74, A61P 1/00, A61P 31/00, A61K 31/00

(54) **Probiotic formulations**

(71) Applicant: Bracco Imaging S.p.A, 20134 Milano (IT)
(72) Inventor: De Haen, Christoph, 20097, San Donato Milanese (IT); Gozzini, Luigia, 20097, San Donato Milanese (IT)
(74) Representative: Poletti, Marco

(57) **Abstract**

The present invention relates to probiotic compositions and kits comprising live bacteria belonging to the natural flora of the human body cavity like intestine and vaginal tract, in particular strains of lactobacillus or bifidobacterium species, and small-molecular-weight non-proteinaceous iron chelators capable of lowering the iron concentration over the whole physiological pH-range of relevance to levels that inhibit growth of pathogens, but which allow for the growth of the bacteria of the composition.

## Description

### Field of the invention

The present invention refers to probiotic and therapeutic formulations and, more in particular, it relates to compositions comprising lactic acid bacteria or bifidobacteria with small-molecular-weight non-proteinaceous iron-chelators, useful for the treatment of infections of the human body cavities.

### Background of the invention

Infections of human body cavities such as, for example, the female genital tract and the intestine, are widely spread pathological conditions known to affect, even recurrently, the majority of population.
Antibiotics are often used to combat these pathological conditions despite the fact that their excessive use may contribute, in a highly regrettable and dramatic way, to the emergence of antibiotic-resistant pathogenic bacterial strains.
As this emergence may pose a serious risk to humanity, it is highly desirable to develop products for the therapy of infections of the body cavities that are not based on antibiotics and, hence, do not lead to the development of antibiotic resistance.

Body cavities including, for example, the vaginal tract, the male urethra, the intestine and the buccal cavity, are all known to be naturally colonized by probiotic bacteria, for instance lactic acid bacteria and bifidobacteria. The normal flora of the vagina and of the gastrointestinal tract consists of a wide variety of genera and species, both anaerobic and aerobic, dominated by the facultative microaerophilic anaerobic genus Lactobacillus (Ref. 1, 2 and 8). These species are known to defend the mucosal surfaces from colonization by pathogenic microorganisms such as toxigenic bacteria and yeasts.
In this respect, the so-called probiotic approach to health maintenance and therapy consists, essentially, in delivering to body cavities, which in healthy individuals are inhabited by commensal microorganisms, the probiotic bacteria with the intent of fostering or reconstituting the natural environment.
To this extent, as commensal microorganisms are known to compete with pathogenic ones, they can have disease preventive or even curative properties.
Many commensal microorganisms have been studied so far but, to date, special attention has been given to various lactobacillus or bifidobacterium species as well as to *Enterococcus faecium* SF68 and the yeast *Saccharomyces boulardii.*
Among them, particularly promising appear to be lactobacillus and bifidobacterium species.
Lactic acid bacteria and bifidobacteria are natural hosts of the intestines and the vagina, where they protect the tissue from pathogenic organisms that, by adhering to the mucosa and tissues, may invade body cavities.
In fact, it has been shown that *Lactobacillus paracasei* strains CNCM 1-1390 and CNCM I-1391 and *Lactobacillus acidophilus* strain CNCM I-1447, which were isolated from healthy babies, bind in large numbers to both buccal and intestinal epithelial cells (Ref. 29), thus demonstrating that they naturally adhere to the same mucosal cells as it may occur for pathogenic microorganisms.
As such, a competition for binding sites between pathogenic microbes and healthy lactic acid bacteria, within body cavities, has been demonstrated (Ref. 30).
Some lactic acid bacteria, in addition, proved to inhibit growth of pathogens. Among them are, as an example, *Lactobacillus paracasei* strains CNCM I-1390 and CNCM I-1391 and *Lactobacillus acidophilus* strain CNCM 1-1447 that inhibited, *in vitro,* growth of enterotoxigenic *E. coli* ATCC 35401 or *Salmonella enteritidis* IMM 2.
Moreover, a mixture of these lactobacillus strains resulted particularly effective as it inhibited, though weakly, even *Vibrio cholerae* El Tor (Ref. 31).

Because all of the above, pharmaceutical products containing lactic acid bacteria or bifidobacteria, for the prevention or treatment of pathological infections, have been already described.
Among them are, just as an example, vaginal capsules comprising a strain of *Lactobacillus gasseri;* lactobacillus vaginal suppositories to prevent recurrence of urinary tract infections after antibiotic therapy (Ref. 3); vaginal medicaments based preferably on *Lactobacillus crispatus* CTV-05 effective against a variety of pathogens (Ref. 4).

Various other products also intended for oral administration and containing live lactic acid bacteria or bifidobacteria are known in the art and recommended, for instance, in the treatment of diarrhea. These products may come as pharmaceutical formulations as well as in the form of fermented milk products.
However, although disease prevention and/or therapy with commensal lactic acid bacteria and bifidobacteria have shown some efficacy (Ref. 5 to 12), the evidence was never sufficiently convincing to lead to a widely spread standard form of treatment.
Presumably, this is because they are not yet as effective as one could expect, at least on a theoretical basis.

It is known in the art that iron is an essential growth factor for virtually every cell and microorganism. The unsatisfactory therapeutic results that have been obtained with previous products comprising commensal microorganisms have been thus associated with too elevated concentrations of free iron (III) ions, which promote the growth of pathogens while disfavoring a number of lactic acid bacteria.
Lactoferrin (see The Merck Index, XIII Ed., 2001, No. 9647), a glycoprotein endogenously produced by neutrophils and also known to be a major component of secreted fluids, including saliva, gastric juice and bile, is a very important factor of the human milk bacteriostatic system.
Because of its iron chelating properties, the inclusion of lactoferrin, either per se or in association with other organic components, is widely known in the art in dietary supplements (Ref. 14).
Lactoferrin capsules may thus contain, as an example, the protein with a degree of purity up to 95% and in quantities up to 480 mg.
However, in preliminary therapeutic trials with very small numbers of patients, only weak indications of some antibacterial (Ref. 15) and antiviral efficacy (Ref. 16) of lactoferrin based products were obtained and the results were insufficient to fostering further studies on this approach.
Some combinations of lactic acid bacteria with lactoferrin are also commercially available (e.g. Colostrum with Lactoferrin Chewable Tablets, Peak Nutrition Inc., Syracuse NY). In this product, however, the quantity of live bacteria (≤ 3.4 x 10⁶ CFU) is orders of magnitude below the limit required for effective intestinal colonization.
Moreover, to the extent of our knowledge, there are no animal or clinical studies demonstrating the efficacy of the combination of lactic acid bacteria with lactoferrin over the bacteria alone.
Lactoferrin has been also suggested to have multiple biological roles including those of facilitating iron absorption, modulating the immune response, regulating embryonic development and influencing cell proliferation. In addition, it has also been demonstrated its role in regulating the release of tumor necrosis factor alpha and interleukin 6 (Ref. 17).
Oral lactoferrin may thus produce many different effects than simple sequestration of iron ions.
Remarkably, it has been demonstrated that certain pathogens can even utilize lactoferrin as an iron source (Ref. 18), thereby counteracting the intended purpose of withholding iron ions from bacteria. For these reasons, the possible therapeutic use of lactoferrin remains a very questionable choice if solely chelation or sequestration of iron ions is intended to be associated with probiotic bacteria.
A small-molecular-weight natural chelator for iron, namely deferoxamine (see The Merck Index, XIII Ed., 2001, No. 2879), has been used experimentally to study the mechanisms of bacterial iron transport and its participation in the competition of commensal lactic acid bacteria with the pathogen *Neisseria gonorrhoeae,* in the mouse genital tract (Ref. 19). It was observed that "the degree of lactobacillus growth on base agar with and without deferoxamine was similar" and it was therefore concluded that "commensal lactobacilli may increase the availability of iron to *N. gonorrhoeae* during infection of females, although the exact mechanism by which this occurs is not known". No suggestion regarding the possible use of the chelator in the formulation of therapeutic or probiotic products was ever made. In any case, certain bacteria possess a deferoxamine receptor (Ref. 20). Accordingly, being a natural siderophore, i.e. iron uptake mediator, from *Streptomyces pylosus,* it can act also as siderophore for certain pathogenic bacteria, e.g. *Yersinia enterocolitica* and *Yersinia pseudotuberculosis* (Ref. 21), thus exerting the undesired feature of iron donor.

Small-molecular-weight non-proteinaceous iron chelators have been shown to possess antimicrobial activity on species that require iron.
In particular, ethylenediaminetetraacetic acid (EDTA), diethylenetriaminepentaacetic acid (DTPA), trans-1,2-diaminocyclohexane-*N,N,N',N*'-tetraacetic acid (CDTA) and triethylene-tetraaminehexaacetic acid (TTHA) have been shown to inhibit the growth of *Staphylococcus epidermidis* (Ref. 22). A number of chelators including *R,S-*ethylenediaminedisuccinic acid (R,S-EDDS), have been shown to inhibit growth of *Corynebacterium xerosis* ATCC 7711 (Ref. 23).
Small-molecular-weight non-proteinaceous iron chelators have also been proposed as additives in buccal disinfectants (Ref. 24), as additives in topical deodorant formulations (Ref. 22, 23 and 25), as components of bactericidal compositions for intestinal use (Ref. 26) and, apparently, for topical use (Ref. 27). A catamenial tampon carrying a chelator was also conceived (Ref. 25).
Chelators with selectivity for first transition series elements, which include iron, intended for use in several biomedical applications, including bacterial and fungal replication, are known in the art (Ref. 28).
However, as the aforementioned prior art documents disclose compositions including chelators being intended for their bactericidal or even sterilizing property, those same compositions could not be used for the prevention and/or treatment of infections within human body cavities, as their effect would be detrimental also to the lactic acid bacteria and bifidobacteria being present in the flora of healty individuals.
To the extent of our knowledge, therefore, no products combining lactic acid bacteria or bifidobacteria with small-molecular-weight non-proteinaceous iron chelators are described, or even theoretically suggested, in the prior art.

### Summary of the invention

We have now found that selected iron chelating agents incorporated into pharmaceutical or probiotic formulations comprising live lactic acid bacteria or bifidobacteria permit, unexpectedly, growth of probiotic bacteria while inhibiting growth of pathogenic microorganisms.
Thus, the present invention concerns a product that combines (a) live bacteria belonging to the natural flora of the body cavity considered, preferably strains of lactobacillus or bifidobacterium species, even more preferably such species selected for their elevated tendency to bind to mucosal surfaces, and/or to co-aggregate with pathogens, with (b) small-molecular-weight non-proteinaceous iron chelators capable of lowering the iron concentration over the whole physiological pH-range of relevance to levels that inhibit growth of pathogens, but which allow for the growth of the bacteria of the composition.

### Detailed description of the invention

It is thus a first object of the present invention a pharmaceutical or probiotic composition comprising (a) at least one lactobacillus species and strain or at least one bifidobacterium species and strain, or mixtures thereof, and (b) at least one small-molecular-weight non-proteinaceous iron chelator.
The compositions of the invention are particularly advantageous as they may be used in the prevention and or treatment of pathologies or pathological states due to infections of the human body cavities.

In the present description, and unless otherwise provided, with the term lactobacillus species and strain and bifidobacterium species and strain we intend those species and strains having good tolerability in humans and affinity for human mucosa.
Preferably, the lactobacillus strain belongs to the species *Lactobacillus johnsonii, Lactobacillus reuterii, Lactobacillus paracasei, Lactobacillus casei, Lactobacillus animalis, Lactobacillus ruminis, Lactobacillus acidophilus, Lactobacillus rhamnosus, Lactobacillus fermentum,* and *Lactobacillus delbrueckii subsp. Lactis.*
Even more preferred lactobacillus strains are those selected from the group consisting of *Lactobacillus johnsonii* Lal NCC 2461 (= CNCM 1-2116), *Lactobacillus reuterii* strains 4000 and 4020 (from BioGaia Biologics Inc., Raleigh, NC), *Lactobacillus paracasei* strains CNCM 1-1390, CNCM 1-1391, CNCM 1-1392, *Lactobacillus casei* strain Shirota, *Lactobacillus acidophilus* strain CNCM 1-1447, *Lactobacillus acidophilus* Lat 11/83, *Lactobacillus acidophilus* NCC 2463 (=CNCM 1-2623), *Lactobacillus rhamnosus* GG (ATCC 53103), *Lactobacillus rhamnosus* 271 (DSMZ 6594), *Lactobacillus rhamnosus* VTT E-800.

As far as the bifidobacterium strain is concerned, it preferably belongs to the species *Bifidobacterium spp., Bifidobacterium bifidum, Bifidobacterium longum, Bifidobacterium pseudolongum, Bifidobacterium infantis, Bifidobacterium adolescentis,* and *Bifidobacterium lactis.*
Even more preferably, bifidobacterium strains are selected from the group consisting of *Bifidobacterium bifidum* NCC 189 (=CNCM 1-2333), *Bifidobacterium adolescentis* NCC 251 (=CNCM 1-2168), *Bifidobacterium lactis* (ATCC 27536), *Bifidobacterium breve* CNCM 1-1226, *Bifidobacterium infantis* CNCM 1-1227, and *Bifidobacterium longum* CNCM 1-1228.
All of these lactobacilli and bifidobacteria are well known and may be isolated according to known methods or obtained from the referred bacterial collections.

Unless otherwise provided, the pharmaceutical or probiotic compositions of the invention may comprise one or more of the lactobacillus species and strain, or one or more of the bifidobacterium species and strain, or even any mixture of the above lactobacilli and bifidobacteria.
Preferably, however, the compositions comprise at least one of the lactobacillus species and strain or at least one of the bifidobacterium species and strain.
Even more preferably, the pharmaceutical or probiotic compositions of the invention comprise at least one of the lactobacillus species and strain.
In the present description, unless otherwise provided, with the term chelator we intend chemical moieties, agents, compounds or molecules, either per se or in the form of pharmaceutically acceptable salts, characterized by the presence of polar groups able to form a complex containing more than one coordinated bond with a transition metal or another metal entity.
In the specific case, the chelator, otherwise known as chelating agent according to the invention, is physiologically acceptable and allows the formation of a complex coordinating iron ions, so as to act as an iron sequestring agent.
Most preferred iron chelators are those which show a conditional formation constant for iron (III) ions over the pH range from 4.6 to 8.2, of at least 10¹⁵ L/mol, and preferably above 10¹⁷ L/mol.
With the term physiologically acceptable we intend any chelator suitable for the administration to humans for the intended therapeutic use, in association with the above lactobacilli and/or bifidobacteria, in any of the possible administration routes.
With the term non-proteinaceous chelator or chelating agent we intend any of the chelators not having the characterizing structure of proteins, being the definition of protein or proteins widely known to the skilled person.
Typically, the non-proteinaceous chelator of the invention has an average molecular weight (MW) lower than 10 kDa, more preferably lower than 5 kDa and even more preferably lower than 1 kDa, that is well below the typical MW associated to protein structures (e.g. lactoferrin MW = 80 kDa).

Suitable chelating agents include those selected from the group consisting of: citric or salicylic acid; pyridinone derivatives such as Deferiprone (see The Merck Index, XIII Ed. 2001, No. 2878), hydroxamates such as Desferroxamine B or acetohydroxamic acid; cathecols such as 1,8-dihydroxynaphthalene-3,6-sulfonic acid, MECAMS, 4-LICAMS, 3,4-LICAMS, 8-hydroxyquinoline or disulfocathecol; polyaminopolycarboxylic acids and derivative thereof comprising, for example, ethylenediamine-N,N'-bis (2-hydroxyphenylacetic acid) (EDDHA), N-(hydroxyethyl)-ethylenediaminetriacetic acid (HEDTA), N,N'-bis(2-hydroxybenzyl)-ethylenediamine-N,N'-diacetic acid (HBED), N,N'-Ethylenebis-2- *(O-Hydroxyphenyl)* Glycine (EHPG), triethylene-tetraaminehexaacetic acid (TTHA); diethylenetriamine pentaacetic acid (DTPA), DTPA-bismethylamide, benzo-DTPA, dibenzo-DTPA, phenyl-DTPA, diphenyl-DTPA, benzyl-DTPA, dibenzyl-DTPA, *N,N*-Bis[2-[(carboxymethyl)[(methylcarbamoyl)methyl]ethyl]-glycine (DTPA-BMA), N-[2-[bis(carboxymethyl)amino]-3-(4-ethoxyphenyl)propyl)]-N-[2-[bis(carboxymethyl) amino]ethyl]glycine (EOB-DTPA), 4-carboxy-5,8,11-tris(carboxymethyl)-1-phenyl-2-oxa-5,8,11-triazatridecan-13**-**oic acid (BOPTA), N,N-bis[2-[bis(carboxymethyl)amino]ethyl]L-glutamic acid (DTPA-GLU) and DTPA-Lys; ethylenediaminotetraacetic acid (EDTA); trans-1,2-diaminocyclohexane; *N,N,N',N'-*tetraacetic acid (CDTA); NTA; PDTA; 1,4,7,10-teraazacyclododecane-1,4,7,-triacetic acid (D03A) and derivatives thereof including, for example, [10-(2-hydroxypropyl)-1,4,7,10-teraazacyclododecane-1,4,7,-triacetic acid (HPD03A) and corresponding [10-(2-hydroxypropyl)-1,4,7,10-tetraazadodecane-1,4,7-triacetato(3-)-*N*¹,*N*⁴,*N*⁷,*N*¹⁰,*O*¹,*O*⁴,*O*⁷,*O*¹⁰]calcinate(1-), calcium (2:1), better known as calteridol or even Ca₃(HPDO3A)₂; 1,4,7-triazacyclononane-N,N',N"-triacetic acid (NOTA); 6-[bis(carboxymethyl)amino]tetrahydro-6-methyl-1H-1,4-diazepine-1,4(5H)-diacetic acid (AAZTA) and derivative thereof; 1,4,7,10-tetraazacyclotetradecane-1,4,7,10-tetraacetic acid (DOTA) and derivatives thereof including, for instance, benzo-DOTA, dibenzo-DOTA, (α,α',α",α"')-tetramethyl-1, 4, 7,10-tetraazacyclotetradecane-1,4,7,10-tetraacetic acid (DOTMA); and 1,4,8,11-tetraazacyclotetradecane-N,N',N",N"'-tetraacetic acid (TETA); or corresponding compounds wherein one or more of the carboxylic groups is replaced by a phosphonic and/or phosphinic group including, for instance, N,N'-bis-(pyridoxal-5-phosphate)-ethylenediamine-N.N'-diacetic acid (DPDP); ethylenedinitrilotetrakis(methylphosphonic) acid (EDTP), 1,4,7,10-tetraazacyclotetradecane-1,4,7,10-tetra(methylenephosphonic) acid (DOTP); as well as texaphyrins, porphyrins and phthalocyanines.
Preferred chelating agents according to the present invention include Deferiprone, HPD03A and derivatives thereof like calteridol, DTPA and derivatives thereof comprising, for instance, DTPA-Glu and DTPA-Lys; DOTA and derivatives thereof; BOPTA; AAZTA and derivatives thereof; EDTA and derivatives thereof; TETA and derivatives thereof.
All of the above chelating agents are widely known in the art or can be prepared according to known methods. For most of them, in addition, there already exists experience with human use.
For a general reference to iron chelators see, for instance, Zu D. Liu, Robert C. Hider; Design of iron chelators with therapeutic application; Coordination Chemistry Reviews Volume 232, Issues 1-2, October 2002, Pages 151-171.
As an example, the iron chelator DTPA in the form of calcium trisodium pentetate (Ditripentat®, Heyl & Co., Berlin, Germany) is used subcutaneously, at doses of 0.5 to 1 g daily for 5 days a week, for the treatment of thalassemia in patients in which deferoxamine caused high-tone deafness (Ref.32).

Additionally, though in the form of a salified gadolinium complex, gadopentetate dimeglumine (Magnevist®, Schering AG, Berlin, Germany) is used as contrast agent for magnetic resonance imaging; its enteral form contains trisodium pentetate as excipient at a level of 455 mg/L of administrable drink.
As the highest recommended dose is 1 L, a maximal oral dose of 455 mg (0.99 mmol) of trisodium pentetate is already being used and proven safe, at least for single administration. The acute oral semilethal dose (*LD*₅₀) of DTPA in mice is 3500 mg/kg. Thus, DTPA is a safe oral drug, and thus constitutes a preferred iron chelator for the compositions of the present invention.
Likewise, the compound 4-carboxy-5,8,11-tris(carboxymethyl)-1-phenyl-2-oxa-5,8,11-triazatridecan-13-oic acid (BOPTA) has been found to have an acute oral *LD*₅₀ in mice of 8.4 mmol/kg. It thus constitutes another prefeered iron chelator for the compositions of the invention.
For pharmaceutical uses these chelators may be also formulated as calcium complexes and neutral salts thereof, since calcium binding is weak enough not to substantially interfere with the iron binding of pharmacological interest.
In this respect, another preferred iron chelator for which there exists experience in man, in the form of a calcium complex, is calteridol also known as Ca₃(HPD03A)₂ and corresponding to [10-(2-hydroxypropyl)-1,4,7,10-tetraazadodecane-1,4,7-triacetato(3-)-*N*¹,*N*⁴,*N*⁷,*N*¹⁰,*O*¹,*O*⁴,*O*⁷,*O*¹⁰]calcinate(1-), calcium (2:1), which is used in the intravenous contrast agent formulations of Gadoteridol (see The Merck Index, XIII Ed., 2001, No. 4353).

The preferred therapeutic or probiotic compositions of the invention can be formulated in different ways, depending on the desired route of administration, following the methods adopted in the pharmaceutical field.
Preferably, the compositions of the invention can be administered either orally or topically, as reported in more details below.
As an example, said compositions can be formulated as a mixture of components or, alternatively, they can equally be offered as separate pharmaceutical formulations in a single kit, for the simultaneous or sequential oral or vaginal administration.

Therefore, it is an additional embodiment of the invention a kit of parts wherein a first part comprises at least one lactobacillus species and strain or at least one bifidobacterium species and strain, or mixtures thereof, and a second part comprises at least one small-molecular-weight non-proteinaceous iron chelator.
Non limitative examples of particularly preferred compositions of the invention are disclosed below.

### Compositions for intestinal use

A first embodiment of the invention is represented by the compositions intended for gastrointestinal use, to be preferably administered as a drink, a capsule, an infant formula or even as a dairy product.
In such a case, the selected bacterial strains may be used so that the amount of bacteria available to the individual corresponds to about 10³ to about 10¹⁴ CFU per day, preferably from about 10⁷ to about 10¹² CFU per day, and even more preferably from about 10⁹ to about 10¹² CFU per day.
The corresponding amount of iron chelator may range from about 10⁻³ to about 10⁻⁹ mol, and preferably from 10⁻⁴ to about 10⁻⁷ mol.
In case the compositions of the invention are intended in the form of an oral formulation, they may be offered as a milk drink, a yoghurt-similar milk product, a cheese, an ice-cream, a fermented cereal-based product, a milk-based powder, an infant formula, a tablet, a capsule, a liquid suspension, a dried oral grit or powder, a wet oral paste or jelly, a grit or powder for dry tube feeding or a fluid for wet tube feeding.
Alternatively, the drink may be prepared before use from a dissolvable capsule containing the active ingredients.
Preferably, the drink may be prepared before use by reconstituting a dry powder containing the lyophilized bacteria and the iron chelator or, alternatively, by reconstituting a dry powder containing the lyophilized bacteria with a physiological solution already comprising the chelator.
The dry powder is preferably packaged into airtight and light-tight sachets, under air or nitrogen, under a noble gas or even under vacuum.
As far as the capsules are concerned, they may be all manufactured according to conventional methods.

From all of the above, it is clear to the skilled person that the compositions of the invention may further comprise any additional excipient among those commonly employed in pharmaceutical forms, for instance to stabilize the formulations themselves, to render them easily dispersible and to give them an agreeable taste.
Among them are, as an example, inulin, fructose, starch, xylo-oligosaccharides, silicon oxide, buffering agents as well as flavors.
Furthermore, optional active ingredients may be also present in the compositions of the invention such as, for instance, vitamins, amino acids and polypeptides.
An example of the optional active ingredient is represented by glutamine (Ref. 33) that, being known as the natural fuel of intestinal mucosal cells, may help intestinal cells to defend themselves under stress by pathogenic organisms (Ref. 34 and 35).
Alanyl-glutamine (Ref. 36) as well as a variety of vitamins may all represent additional ingredients within the instant compositions.
The presence of transition metals should be preferably avoided so as not to impair the binding/sequestration of the naturally occurring iron ions by the chelator. However, by considering that the preferred chelators according to the invention bind iron ions much stronger than other physiological transition-metal ions, for instance zinc or copper, the presence of these latter does not affect the efficacy of the instant compositions.

### Compositions for vaginal use

According to an additional embodiment, the present invention also provides for a composition intended for the vaginal use, for instance as a compressed vaginal suppository or insert, preferably of the rapidly dissolving type, as a tampon or as a douche.
Vaginal suppositories and capsules are well-known pharmaceutical formulations. During their manufacturing process, however, special care should be taken to operate at temperature conditions at which the bacteria may survive, according to methods known in the art (Ref. 4).
Vaginal inserts are also known in the art and may be produced, for instance, by powder compression of maltodextrin beads including the components of the invention (Ref. 4). Standard catameneal tampons, and their production methods, can be well adapted for obtaining vaginal tampons bearing the ingredients of the invention on their surface; preferably, the final tampon needs to be packaged to protect it from moisture.
Vaginal douches are commercially known and consist of a product to be locally applied through a proper applicator, hence suitable for vaginal delivery of the compositions of the invention.
Clearly, unlike otherwise provided, also the compositions intended for vaginal use may comprise additional excipients among those known in the art (e.g. buffering agents) and/or active ingredients known for formulations of this type.
The compositions of the invention resulted to be particularly effective in the colonization of the gastrointestinal tract or the vaginal tract and, hence, allow for the restoration of a well functioning microflora, particularly in the case of a previous use of antibiotics.
As such, they may find a wide range of applications either in the maintenance of probiotic bacteria adhering to healthy mucosal surfaces as well as in the treatment of the infections of the human body cavities such as the vaginal tract, the male urethra, the intestine and the buccal cavity.
Vaginal infections wherein the compositions of the invention may be advantageously used may comprise, as a non limiting example, bacterial vaginosis, symptomatic yeast vaginitis, gonorrhea, chlamydia, trichomoniasis, human immunodeficiency virus infection, urinary tract infection or pelvic inflammatory disease.
Among the pathological conditions of the gastrointestinal tract, instead, the compositions of the invention may be used for the treatment of acute diarrhea in adults and infants, rotavirus-related, travel's or antibiotic-associated diarrhea, and recurrent *clostridium difficile* colitis.

### Experimental section

With the aim of illustrating the present invention, without posing any limitation to it, the following examples are now given.

### Example 1

### Drink formulation

A powder containing lactobacilli and at least one small molecular weight non-proteinaceous iron chelator, suitable for preparing a drink, is formulated to have the following composition:

| | |
|---|---|
| Inulin | 145.00 kg |
| Fructose | 57.69 kg |
| L-glutamine | 50.00 kg |
| Xylo-oligosacchrides | 25.00 kg |
| *Lactobacillus paracasei* CNCM I-1390 | 11.13 kg |
| (3.85x10¹¹ CFU/g) | |
| Orange aroma | 10.50 kg |
| Silicon dioxide | 0.40 kg |
| Pentetate calcium trisodium (DTPA CaNa₃) | 0.21 kg |
| Vitamin B6 hydrochloride | 0.07 kg |
| Lot | 300.00 kg |

Portions of 7 g of this powder are filled into sachets under low humidity conditions and sealed. A single dose of the drink consists of the content of a sachet suspended in a glass of water.

### Example 2

### Drink formulation

Analogously to Example 1, a powder containing the selected strain of lactobacilli and the small molecular weight non-proteinaceous iron chelator is formulated, wherein the chelator is calteridol, which is [10-(2-Hydroxypropyl)-1,4,7,10-tetraazadodecane-1,4,7-triacetato(3-)-*N*¹,*N*⁴*,N*⁷*,N*¹⁰*,O*¹*,O*⁴*,O*⁷*,O*¹⁰]calcinate(I-), calcium (2: 1), abbreviated Ca₃(HP-DO3A)₂, in the same amount.

### Example 3

### Drink formulation

A powder containing the selected lactobacilli strain and at least one small molecular weight non-proteinaceous iron chelator suitable for preparing a drink is formulated to have the following composition:

| | |
|---|---|
| Corn starch | 86.24 kg |
| Fructose | 120.00 kg |
| L-glutamine | 42.87 kg |
| Xylo-oligosacchrides | 30.00 kg |
| *Lactobacillus paracasei* CNCM I-1390 | 11.13 kg |
| (3.85x10¹¹ CFU/g) | |
| Orange aroma Drycell 01142 | 9.00 kg |
| Silicon dioxide | 0.33 kg |
| Deferiprone (1,2-dimethyl-3-hydroxypyrid-4-one) | 0.43 kg |
| Lot | 300.00 kg |

Portions of 7 g of this powder are filled into sachets under low humidity conditions and sealed. A single dose of the drink consists of the content of a sachet suspended in a glass of water.

### Example 4

### Therapeutic infant formulation

A therapeutic infant formulation is obtained by mixing from 0.5% to 5%, preferably 2%, of polypeptides; from 0.2% to 10%, preferably 4%, of fat; from 1 % to 25%, preferably 8%, of non-levan carbohydrates (including lactose 65%, maltodextrin 20% and starch 15%); a proper amount of an iron chelator, and at least 10⁶ CFU/mL of the following strain: *Lactobacillus acidophilus* CNCM I-1447, in combination with traces of vitamins to meet the daily requirements; from 0.01% to 2%, preferably 0.3%, of minerals, and from 50% to 75% of water.

### Example 5

### Therapeutic dairy product

A yoghurt-like milk product is prepared by the following procedure. One liter of a milk product containing 2.8% of fats and supplemented with 2% of skimmed milk powder and 6% of sucrose is prepared. Then, it is pasteurized at 96°C for 30 min according to known methods. A proper amount of calteridol is then added.
A preculture of *Lactobacillus paracasei* CNCM 1-1390 is reactivated in a medium containing 10% of reconstituted milk powder and 0.1 % of commercial yeast extract with 1% sucrose. The pasteurized milk product is then inoculated with 1% of the reactivated preculture and this milk product is then allowed to ferment until the pH reaches a value of 4.5. The resulting therapeutic yoghurt-like milk-product is stored at 4°C.

### Example 6

### Vaginal suppository

In a sterilized blender, 0.12 kg finely ground calteridol is blended with 1.33 kg of polyethylene glycol 1000 (PEG 1000) under nitrogen, during which the polyethylene glycol melts. Under continued mild mixing the calteridol-PEG 1000 mixture is cooled until it has returned to a semi-solid consistency.
By intensive mechanical mixing, under vacuum in a cooled container, the following ingredients are thus admixed:

| | |
|---|---|
| Polyethylene glycol 1000 | 72.43 kg |
| Polyethylene glycol 4000 | 24.48 kg |
| *Lactobacillus paracasei* CNCM 1-1390 | 2.00 kg |
| (3.85x10¹¹ CFU/g) | |
| Calteridol-PEG 1000 mixture | 1.09 kg |
| Lot | 100.00 kg |

The resulting mixture is formed into 5 g suppositories by cooled compression molding.

### Example 7

### Vaginal capsules

The procedure substantially follows the one of Example 3 of Ref. 4, with the main difference that the maltodextrin beads is first sprayed with an aqueous solution of calteridol sodium and dried in the fluid bed drier, and then sprayed with the bacterial cell matrix suspension.
A preservation matrix is prepared as follows: 2 parts gelatin (e.g. 137.5 g per 500 mL reagent water) and 4 parts skim milk (e.g. 15 g per 250 mL reagent water) is autoclaved at about 121°C for about 15 min. 4 parts xylitol (e.g. 59 g per 250 mL reagent water) and 4 parts dextrose (25 g per 250 mL reagent water) are mixed together, adjusted to pH 7.2-7.4 and filter sterilized with a 0.22 µm filter. The sterile components are then combined into a single solution (gelatin base) and stored at 2-8°C. Ascorbic acid is prepared as a 5% (w/w) solution, filter-sterilized with a 0.22 µm filter and stored at - 20°C. At the time of production of the vaginal medicant, the gelatin base is melted and tempered to about 35°C. Then, the 5% (w/w) ascorbic acid is added to the gelatin base at a ratio of 1:10 to form the preservation matrix solution.
A solution of calteridol (462 mg/mL reagent water) is prepared and sterilized at 121°C for 20 min.
Lactobacillus paracasei CNCM I-1390 is grown as described in Ref. 30 to a cell density of about 5 x 10⁹ cells/mL and a cell pellet is prepared by centrifugation for 5 min at 1400-1600 rpm.
The cell pellet is resuspended in phosphate-buffered saline and again pelleted by centrifugation. The cell pellet is resuspended in 1 part phosphate-buffered saline and 10 parts preservation matrix solution. The cell matrix suspension is mixed gently and thoroughly and maintained at 35°C with continuous mixing.
To form the complete vaginal medicant, a fluid bed dryer having sterilized components is assembled for use. Maltodextrin beads (Maltrin® QD M510, Grain Processing Corporation, Muscatine, Iowa) are placed into the fluid bed dryer and are dried at 33°C until sufficiently dry. The air pressure is then set to 14 psi, and the solution of calteridol sodium [Ca₃(HP-DO3A)₂] (50 mL per kg of maltodextrin beads) is sprayed onto the beads using a peristaltic pump. The beads are allowed to dry for 30 min at about 38°C.

The temperature is returned to 33°C and the cell matrix suspension (50 mL per kg of maltodextrin beads) is sprayed onto the beads with the help of the peristaltic pump. After 50% of the cell matrix suspension has been sprayed onto the beads, the heat is increased to 38°C. After all the cell matrix suspension has been sprayed onto the beads, the coated beads are allowed to dry at about 38°C for about 30 min. The coated maltodextrin beads can be frozen and stored as a powder.
The powder is filled into gelatin capsules Type 00 to a level of about 500 mg per capsule. One capsule contains about 5 x 10⁸ CFU of lactobacilli.
The capsules can be packaged, optionally under nitrogen or vacuum, into air and vapor-tight primary packaging material.

### Example 8

### Vaginal capsules

The procedure substantially follows the one of the preceding Example 7, with the main difference that the phosphate-buffered saline used in the preparation of the cell matrix suspension is modified to contain 10 mM of a small molecular weight non-proteinaceous iron chelator, preferably calteridol sodium [Ca₃(HP-DO3A)₂], under reduction of the sodium chloride concentration to achieve isotonicity, i.e. about 290 mOsmol/kg.

### Example 9

### Vaginal capsules

Vaginal capsules are prepared essentially as described in present Example 8, except that *Lactobacullus paracasei* CNCM 1-1390 is replaced by the *Lactobacullus crispatus* CTV-05 described in Ref. 4.

### Example 10

### Vaginal insert

The maltodextrin beads coated with *Lactobacullus paracasei* CNCM 1-1390 are prepared as described in Example 14. Vaginal inserts are prepared by compression.

### Example 11

### Vaginal tampon

A lyophilized powder containing lactic acid bacteria, the chelator and the excipients is prepared.
A vaginal tampon is prepared composed of an absorbent compressed, cylindrical core of tissue pulp and short rayon fibers. Maximal dryness of the core was assured by placing it in a high vacuum overnight and working in a low humidity environment. The tailing one-third is temporarily wrapped with plastic and the leading two-thirds are covered with the described powder by turning and rubbing it by hand on a flat glass surface. A non-woven cover is wrapped around the core and a withdrawal string is knotted around the core at its trailing end. The finished tampon is packaged under dry nitrogen into airtight and light-tight pharmaceutical sachet.

### Bibliography

1. Gorbach S.L., Menda K.B., Thadepalli H. & Keith L.: Anaerobic microflora of the cervix in healthy women. Am. J. Obstet. Gynecol. 117, 1053-1055, 1973.
2. Redondo-Lopez V., Cook R.L. & Sobel J.G.: Emerging role of lactobacilli in the control and maintenance of the vaginal bacterial microflora. Rev. Infect. Dis. [Chicago]. 12, 856-872, 1990.
3. Reid G., Bruce A.W. & Taylor M.: Influence of three-day antimicrobial therapy and lactobacillus vaginal suppositories on recurrence of urinary tract infection. Clin. Ther. 14, 11-16, 1992.
4. Chrisope G.L.: Vaginal *Lactobacillus* medicant. US6468526 B2 (Priority Date Dec. 21, 2001) 2002.
5. Bin L.X.: Controlled clinical trial of Lacteol fort sachets versus furazolidone or berberine in the treatment of acute diarrhea in children. Ann. Pédiatr. [Paris], 42, 396-401, 1995.
6. Boulloche J., Mouterde O. & Mallet E.: Management of acute diarrhea in infants and young children. Ann. Pédiatr. [Paris], 41, 1-7, 1994.
7. Biller J.A., Katz A.J., Flores A.F., Buie T.M. & Gorbach S.L.: Treatment of recurrent Clostridium difficile colitis with Lactobacillus GG. J. Pediatr. Gastroenterol. Nutr. 21, 224-226, 1995
8. Isolauri E., Kaila M., Mykänen H., Ling W.H. & Salminen S.: Oral bacteriotherapy for viral gastroenteritis. Dig. Dis. Sci. 39, 2595-2600, 1994.
9. Raza S., Graham S.M., Allen S.J., Sultana S., Cuevas L. & Hart C.A.: Lactobacillus GG promotes recovery from acute nonbloody diarrhea in Pakistan. Pedatr. Inf. Dis. J. 14, 107-111, 1995.
10. 11. Saavedra J.M., Bauman N.A., Oung I., Perman J.A. & Yolken R.H.: Feeding of Bifidobacterium bifidum and Streptococcus thermophilus to infants in hospital for prevention of diarrhea and shedding of rotavirus. Lancet 344, 1046-1049, 1994.
11. Hallen A., Jarstrand C. & Pahlson C.: Treatment of bacterial vaginosis with lactobacilli. Sex. Trasm. Dis. 19, 146-148, 1992.
12. Sarkinov S.E., Krymshokalova Z.S., Kafarskaia L.I. & Korshunoov V.M.: [The use of the biotherapeutic agent Zhlemik for correcting the microflora in bacterial vaginosis] Zhur. Mikrobiol. Epidemiol. Immunobiol. [Moscow] (1), 88-90, 2000.
13. Wooldridge K.G. & Williams P.H.: Iron uptake mechanisms of pathogenic bacteria. FEMS microbiol. Rev. 12, 325-348, 1993.
14. Gohlke M.B. & Cockrum R.H.: Dietary supplement combining colostrums and lactoferrin in a mucosal delivery format. US6258383 B1 (Priority Date Aug. 6, 1999) 2001.
15. Trümpler U., Straub P.W. & Rosenmund A.: Antibacterial prophylaxis with lactoferrin in neutropenic patients. Eur. J. Clin. Microbiol. Infect. Dis. 8, 310-313, 1989.
16. Tanaka K., Ikeda M., Nozaki A., Kato N., Tsuda H., Saito S. & Sekihara H.: Lactoferrin inhibits hepatitis C virus viremia in patients with chronic hepatitis C: a pilot study. Jpn. J. Cancer Res. 90, 367-371, 1999.
17. Machnicki M., Zimecki M. & Zagulski T.: Lactoferrin regulates the release of tumour necrosis factor alpha and interleukin 6 in vivo. Int. J. Exp. Pathol. 74, 433-439, 1993.
18. Mickelsen P.A., Blackman E. & Sparling P.F.: Ability of Neisseria gonorrhoeae, Neisseria meningitides, and commensal Neisseria species to obtain iron from lactoferrin. Infect. Immun. 35, 915-920, 1982.
19. Jerse A.E., Crow E.T., Bordner A.N., Rahman I., Cornelissen C.N., Moench T.R. & Mehrazar K.: Growth of Neisseria gonorrhoeae in the female mouse genital tract does not require the gonococcal transferring or hemoglobin receptors and may be enhanced by commensal lactobacilli. Infect. Immun. 70, 2549-2558, 2002.
20. Nelson M., Carrano C.J. & Szaniszlo P.J.: Identification of the ferrioxamine B receptor, Fox B, in Escherichia coli K12. Biometals 5, 37-46, 1992.
21. Desferal® (deferoxamine) package insert. Novartis Pharma AG, Basel, Switzerland, 2000.
22. Johnson P.A., Landa A.S., Makin A. & McKay V.A.: Deodorant products. US 6503490 B2 (Priority Date Oct. 9, 2001) 2003.
23. Bachmann F., Ochs D., Utz R. & Ehlis T.: Use of nitrogen-containing complexing agents for deodorization and antimicrobial treatment of the skin and textile fibre materials. US 2002/0031537 A1, (Priority Date May 22, 1996) 2002.
24. Asami T., Takhashi M, Andrews J.F. & Boettcher E.: Oral disinfectant for companion animals. US5460802 (Priority Date July 18, 1994) 1995.
25. Kraskin K.S.: Inhibiting production of undesirable products on body surfaces and environs employing aminopolycarboxylic compounds. US 4356190 (Priority Date Oct. 16, 1978) 1982.
26. Blackburn P., Projan S.J. & Goldberg E.B.: Pharmaceutical bacteriocin compositions and methods for using the same. US5334582 (Priority Date July 6, 1993) 1994.
27. Bailley G.M. & Hall R.G.: Bactericidal composition. WO 97/02010 (Priority Date July 5 1995) 1997.
28. Winchell H.S., Klein J. Y., Simhon E.D., Cyjon R.L., Klein O. & Zaklad H.: Compounds with chelation affinity and selectivity for first transition series elements, and their use in medical therapy and diagnosis. WO 97/01360 (Priority Dates June 26 and Nov. 20, 1995) 1997.
29. Morelli L., Bottazzi V., Gozzini L. & de Haën C.: Lactobacillus strains of human origin, their compositions and uses thereof. US 5,709,857 (Priority Dates May 26 and Aug. 25; 1994) 1998.
30. Bernet M.F., Brassart D., Neeser J.R. & Servin A.L.: Lactobacillus acidophilus LA1 binds to cultured human intestinal cell lines and inhibits attachment and cell invasion by enterovirulent bacteria. Gut, 35, 483-489, 1994.
31. Drago L., Gismondo M. R., Lombardi A., de Haën C., & Gozzini L.: Inhibition of in vitro growth of enteropathogens by new lactobacillus isolates of human intestinal origin. FEMS Microbiol. Lett. 153, 455-463, 1997.
32. Wonke B., Hoffbrand A.V., Aldouri M., Wickens D., Flynn D., Steams M. & Warner P.: Reversal of desferrioxamine induced auditory neurotoxicity during treatment with Ca-DTPA. Arch. Dis. Child. 64, 77-82, 1989.
33. de Haën C. & Gozzini L.: Pharmaceutical and diet formulations for the prophylaxis and treatment of gastrointestinal disorders. US 6007808 (Priority Date June 23, 1995) 1999.
34. Wischmeyer P.E., Musch M.W., Madonna M.B., Thisted R. & Chang E.B.: Glutamine protects intestinal epithelial cells: role of inducible HSP70. Am. J. Physiol. 272 (Gastrointes. Liver Physiol. 35) G879-G884, 1997.
35. Wilmore D.W. & Shabert J.K.: Role of glutamine in immunologic responses. Nutrition 14, 618-626, 1998.
36. Scheppach W., Loges C., Bartram P., Christl S.U., Richter F., Dusel G., Stehle P., Fuerst P. & Kasper H.: Effect of free glutamine and Alanyl-Glutamine dipeptide on mucosal proliferation of the human ileum and colon. Gastroenterology 107, 429-434, 1994.

## Claims

1. A pharmaceutical or probiotic composition comprising (a) at least one lactobacillus species and strain or at least one bifidobacterium species and strain, or mixtures thereof, and (b) at least one small molecular weight non-proteinaceous iron chelator.

2. A composition according to claim 1 wherein said lactobacillus belongs to the species *Lactobacillus johnsonii, Lactobacillus reuterii, Lactobacillus paracasei, Lactobacillus casei, Lactobacillus animalis, Lactobacillus ruminis, Lactobacillus acidophilus, Lactobacillus rhamnosus, Lactobacillus fermentum,* and *Lactobacillus delbrueckii subsp. Lactis.*

3. A composition according to claim 2 wherein the lactobacillus strains are selected from the group consisting of *Lactobacillus johnsonii* La1 NCC 2461 (= CNCM I-2116), *Lactobacillus reuterii* strains 4000 and 4020 (from BioGaia Biologics Inc., Raleigh, NC), *Lactobacillus paracasei* strains CNCM I-1390, CNCM I-1391, CNCM I-1392, *Lactobacillus casei* strain Shirota, *Lactobacillus acidophilus* strain CNCM I-1447, *Lactobacillus acidophilus* Lat 11/83, *Lactobacillus acidophilus* NCC 2463 (=CNCM 1-2623), *Lactobacillus rhamnosus* GG (ATCC # 53103), *Lactobacillus rhamnosus* 271 (DSMZ 6594), *Lactobacillus rhamnosus* VTT E-800.

4. A composition according to claim 1 wherein said bifidobacterium belongs to the species *Bifidobacterium spp., Bifidobacterium bifidum, Bifidobacterium longum, Bifidobacterium pseudolongum, Bifidobacterium infantis, Bifidobacterium adolescentis,* and *Bifidobacterium lactis.*

5. A composition according to claim 4 wherein the bifidobacterium strains are selected from the group consisting of *Bifidobacterium bifidum* NCC 189 (=CNCM I-2333), *Bifidobacterium adolescentis* NCC 251 (=CNCM 1-2168), *Bifidobacterium lactis* (ATCC 27536), *Bifidobacterium breve* CNCM I-1226, *Bifidobacterium infantis* CNCM I-1227, and *Bifidobacterium longum* CNCM I-1228.

6. A composition according to claim 1 wherein the iron chelator has a formation constant for iron (III) ions, over the pH range from 4.6 to 8.2, of at least 10⁵ L/mol.

7. A composition according to claim 1 wherein the iron chelator is selected from the group consisting of: citric or salicylic acid; pyridinone derivatives such as Deferiprone (see The Merck Index, XIII Ed. 2001, No. 2878), hydroxamates such as Desferroxamine B or acetohydroxamic acid; cathecols such as 1,8-dihydroxynaphthalene-3,6-sulfonic acid, MECAMS, 4-LICAMS, 3,4-LICAMS, 8-hydroxyquinoline or disulfocathecol; polyaminopolycarboxylic acids and derivative thereof comprising, for example, ethylenediamine-N,N'-bis (2-hydroxyphenylacetic acid) (EDDHA), N-(hydroxyethyl)-ethylenediaminetriacetic acid (HEDTA), N,N'-bis(2-hydroxybenzyl)-ethylenediamine-N,N'-diacetic acid (HBED), N,N'-Ethylenebis-2- *(O-Hydroxyphenyl)* Glycine (EHPG), triethylene-tetraaminehexaacetic acid (TTHA); diethylenetriamine pentaacetic acid (DTPA), DTPA-bismethylamide, benzo-DTPA, dibenzo-DTPA, phenyl-DTPA, diphenyl-DTPA, benzyl-DTPA , dibenzyl-DTPA, *N,N*-Bis[2-[(carboxymethyl)[(methylcarbamoyl)methyl]ethyl]-glycine (DTPA-BMA), N-[2-[bis(carboxymethyl)amino]-3-(4-ethoxyphenyl)propyl)]-N-[2-[bis(carboxymethyl) amino]ethyl]glycine (EOB-DTPA), 4-carboxy-5,8,11-tris(carboxymethyl)-1-phenyl-2-oxa-5,8,11-triazatridecan-13-oic acid (BOPTA), N,N-bis[2-[bis(carboxymethyl)amino]ethyl]L-glutamic acid (DTPA-GLU) and DTPA-Lys; ethylenediaminotetraacetic acid (EDTA); trans-1,2-diaminocyclohexane; *N,N,N',N'-*tetraacetic acid (CDTA); NTA; PDTA; 1,4,7,10-teraazacyclododecane-1,4,7,-triacetic acid (D03A) and derivatives thereof including, for example, [10-(2-hydroxypropyl)-1,4,7,10-teraazacyclododecane-1,4,7,-triacetic acid (HPD03A) and corresponding [10-(2-hydroxypropyl)-1,4,7,10-tetraazadodecane-1,4,7-triacetato(3-)-*N*¹,*N*⁴,*N*⁷,*N*¹⁰,*O*¹,*O*⁴,*O*⁷,*O*¹⁰]calcinate(1-), calcium (2:1), better known as calteridol or even Ca₃(HPDO3A)₂; 1,4,7-triazacyclononane-N,N',N"-triacetic acid (NOTA); 6-[bis(carboxymethyl)amino]tetrahydro-6-methyl-1H-1,4-diazepine-1,4(5H)-diacetic acid (AAZTA) and derivative thereof; 1,4,7,10-tetraazacyclotetradecane-1,4,7,10-tetraacetic acid (DOTA) and derivatives thereof including, for instance, benzo-DOTA, dibenzo-DOTA, (α,α',α",α"')-tetramethyl-1,4,7,10-tetraazacyclotetradecane-1,4,7,10-tetraacetic acid (DOTMA); and 1,4,8,11-tetraazacyclotetradecane-N,N',N",N"'-tetraacetic acid (TETA); or corresponding compounds wherein one or more of the carboxylic groups is replaced by a phosphonic and/or phosphinic group including, for instance, N,N'-bis-(pyridoxal-5-phosphate)-ethylenediamine-N.N'-diacetic acid (DPDP); ethylenedinitrilotetrakis(methylphosphonic) acid (EDTP), 1,4,7,10-tetraazacyclotetradecane-1,4,7,10-tetra(methylenephosphonic) acid (DOTP); as well as texaphyrins, porphyrins and phthalocyanines.

8. A composition according to claim 7 wherein the iron chelator is selected from Deferiprone, HPD03A and derivatives thereof including Calteridol, DTPA and derivatives thereof including DTPA-Glu and DTPA-Lys, DOTA and derivatives thereof, BOPTA, AAZTA and derivatives thereof, EDTA and derivatives thereof, TETA and derivatives thereof.

9. A composition according to claim 8 wherein the iron chelator is selected from Deferiprone, Calteridol, BOPTA and DTPA.

10. A composition according to claim 1 for intestinal use in the form of a milk drink, a yoghurt-similar milk product, a cheese, an ice-cream, a fermented cereal-based product, a milk-based powder, an infant formula, a tablet, a capsule, a liquid suspension, a dried oral grit or powder, a wet oral paste or jelly, a grit or powder for dry tube feeding or a fluid for wet tube feeding.

11. A composition according to claim 1 for vaginal use in the form of a compressed vaginal suppository or insert, tampon or douche.

12. A composition according to any one of claims 10 or 11 wherein the amount of bacterial strains provides for from 10³ to 10¹⁴ CFU/day.

13. A kit of parts wherein a first part comprises at least one lactobacillus species and strain or at least one bifidobacterium species and strain, or mixtures thereof, and a second part comprises at least one small-molecular-weight non-proteinaceous iron chelator.

14. Use of a composition according to anyone of claims from 1 to 12 or of a kit according to claim 13 for the prevention or treatment of the infections of the human body cavities including the vaginal tract, the male urethra, the intestine and the buccal cavity.

15. Use of a composition according to any one of claims from 1 to 12 or of a kit according to claim 13 for the treatment of bacterial vaginosis, symptomatic yeast vaginitis, gonorrhea, chlamydia, trichomoniasis, human immunodeficiency virus infection, urinary tract infection or pelvic inflammatory disease, acute diarrhea in adults and infants, rotavirus-related, travel's or antibiotic-associated diarrhea, and recurrent *Clostridium difficile* colitis.
